# EUROPEAN PATENT APPLICATION

(11) **EP 4 008 303 A1**
(43) Date of publication of application: **08.06.2022**
(21) Application number: 20850810.1
(22) Date of filing: 28.07.2020
(51) Int. Cl.: A61K 8/02, A61Q 1/02, A61Q 1/10

(54) **SOLID COSMETIC PREPARATION AND METHOD FOR PRODUCING SAME**

(30) Priority: 02.08.2019 JP 2019143060
(71) Applicant: Shiseido Company, Ltd., Chuo-ku Tokyo 104-0061 (JP)
(72) Inventor: TAKAHASHI, Kiyoshi, Tokyo 104-0061 (JP); OIKAWA, Hiromi, Tokyo 104-0061 (JP); KINO, Yoshitaka, Tokyo 104-0061 (JP); NISHIMI, Kazuhiro, Tokyo 104-0061 (JP)
(74) Representative: Ter Meer Steinmeister & Partner
(86) International application number: PCT/JP2020/028854
(87) International publication number: WO 2021/024854

(57) **Abstract**

[Objective]

To obtain a solid cosmetic having superior product appeal.

[Constitution]

A solid cosmetic (1) which is held in a tray (2) includes: a region of fine recesses and protrusions (1c) with differences in height within a range from 0.1 to 10 mm and that includes inclined surfaces formed on an upper surface (1a) of the solid cosmetic (1); and a colorant (3) with a primary particle diameter within a range from 10 to 100 µm added to a portion of the upper surface (1a) of the solid cosmetic (1) that includes at least a portion of the region of fine recesses and protrusions (1c) by dispersive adhesion.

## Description

### Technical Field

The present disclosure is related to a solid cosmetic, and more particularly to a solid cosmetic, to which a colorant is added by dispersive adhesion as a decorative element, as well as a method for producing the solid cosmetic.

### Background Art

Conventionally, adding various decorative elements on the surfaces of solid cosmetics such as eye shadow, foundation, and face powder has been proposed, in order to increase eye catching properties and product appeal. Patent Documents 1 through 3, for example, disclose that powders, particularly a glossy powder such as a pearly agent and a lame, for example, be added to the surfaces of solid cosmetics by dispersive adhesion.

### Patent Literature

[Patent Document 1]
   Japanese Unexamined Patent Publication No. H1-146812
[Patent Document 2]
   Japanese Patent No. 4027868
[Patent Document 3]
   Japanese Patent No. 5139636

### Summary of the Invention

### Technical Problem

Adding such powders or particles by dispersive adhesion as decorative elements is effective in increasing the eye catching properties of solid cosmetics, but room for improvement remains. Therefore, an objective of the present invention is to provide a solid cosmetic that exhibits greater product appeal than conventional products. In addition, another objective of the present invention is to provide a method for producing such a solid cosmetic.

### Solution to the Problem

The solid cosmetic of the present invention is a solid cosmetic which is held in a tray, characterized by:
a region of fine recesses and protrusions with differences in height within a range from 0.1 to 10 mm and that include inclined surfaces being formed on an upper surface of the solid cosmetic; and
a colorant with a primary particle diameter within a range from 10 to 100 µm is added to a portion of the upper surface of the solid cosmetic that includes at least a portion of the region of fine recesses and protrusions by dispersive adhesion. Note that the above "inclined surfaces" refer to surfaces which are not parallel to portions of the solid cosmetic at which a raised portion and recesses are not formed.

It is desirable for the colorant to be a glossy powder such as a pearly agent or a lame.

It is desirable for a raised portion having a predetermined pattern to be formed on the upper surface of the solid cosmetic, and for the region of fine recesses and protrusions to be formed at least on a portion of a surface of the raised portion.

It is desirable for a recessed portion having a predetermined pattern to be formed on the upper surface of the solid cosmetic, and for the region of fine recesses and protrusions to be formed at least on a portion of a surface of the recessed portion.

The raised portion and the recessed portion may be formed on the upper surface of the solid cosmetic, and the region of fine recesses and protrusions may be formed at least on a portion of the surfaces of the raised portion and the recessed portion.

The upper surface of the solid cosmetic of the present invention may be formed to be flat, and the region of fine recesses and protrusions may be formed on the flat upper surface.

The upper surface of the solid cosmetic of the present invention may be sectioned into a plurality of regions, and each of the plurality of regions of the solid cosmetic may be colored to be different colors.

In the case that the upper surface of the solid cosmetic is sectioned into a plurality of regions as described above, it is desirable for the region of fine recesses and protrusions to be provided over the plurality of regions.

The method for producing a solid cosmetic of the present invention is a method for producing the solid cosmetic of the present invention described above, and is characterized by comprising:
placing a solid cosmetic having a region of fine recesses and protrusions with differences in height within a range from 0.1 to 10 mm and that include inclined surfaces an upper surface thereof on a tray, and thereafter;
dispersing a composition that contains a colorant with a primary particle diameter within a range from 10 to 100 µm onto at least the region of fine recesses and protrusions formed on the upper surface of the solid cosmetic.

In the method for producing a solid cosmetic, it is desirable for the composition to be dispersed onto the upper surface of the solid cosmetic via a mask having an opening that corresponds to a range onto which the colorant is to be dispersively adhered. It is preferable for two or more recessed portions and/or protruding portions to be present within the range onto which the colorant is to be dispersively adhered. The number of recesses and protrusions is directly linked to the complexity of adding decorative elements by shaping. A solid cosmetic on which decorative elements are added in a complex manner will have a more attractive outer appearance to consumers.

### Advantageous Effects of the Invention

In the solid cosmetic according to the present invention, the region of fine recesses and protrusions has differences in height within a range from 0.1 to 10 mm and includes inclined surfaces, and the colorant which is dispersively adhered on the region of fine recesses and protrusions has a primary particle diameter within a range from 10 to 100 µm. Therefore, the colorant can be an ink in which the colorant is stably dispersed in a solvent.

In addition, the method for producing a solid cosmetic according to the present invention comprises placing a solid cosmetic having a region of fine recesses and protrusions with differences in height within a range from 0.1 to 10 mm and that include inclined surfaces an upper surface thereof on a tray, and thereafter;
dispersing a composition that contains a colorant with a primary particle diameter within a range from 10 to 100 µm onto at least the region of fine recesses and protrusions formed on the upper surface of the solid cosmetic. Therefore, it is possible to produce the solid cosmetic according to the present invention.

### Brief Description of the Drawings

FIG. 1 is a perspective view that illustrates a solid cosmetic according to a first embodiment of the present invention.
FIG. 2 is a schematic cross sectional view that illustrates a portion of the solid cosmetic.
FIG. 3 is a schematic diagram that illustrates a method for producing the solid cosmetic.
FIG. 4 is a perspective view that illustrates a solid cosmetic according to a second embodiment of the present invention.
FIG. 5 is a perspective view that illustrates a solid cosmetic according to a second embodiment of the present invention.

### Embodiments of the Invention

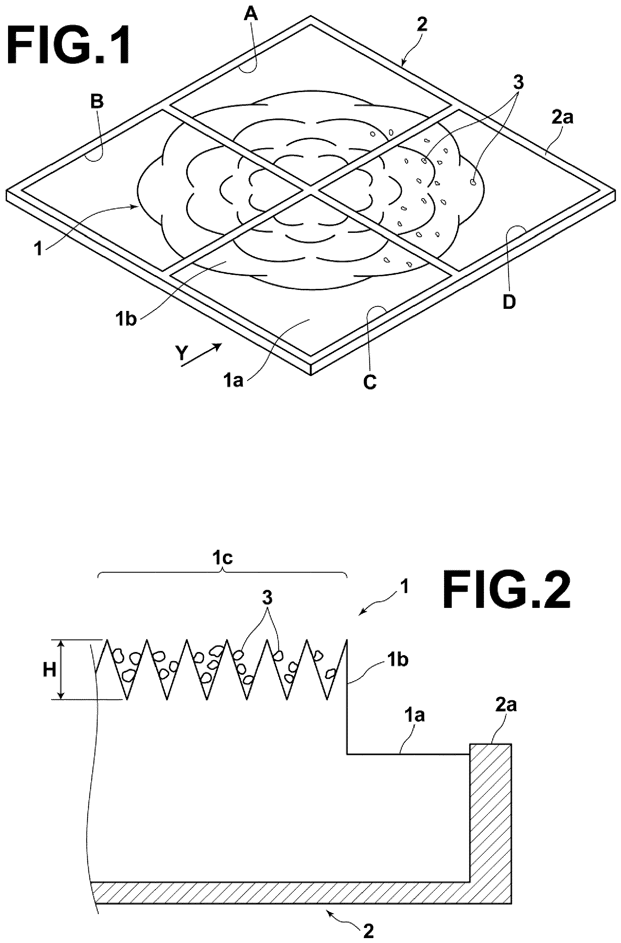

Hereinafter, embodiments of the present invention will be described with reference to the attached drawings. FIG. 1 is a perspective view that illustrates a solid cosmetic 1 according to a first embodiment of the present invention. The solid cosmetic 1 is an eye shadow, for example, and is held on a comparatively thin tray 2. The tray 2 is sectioned into four regions A, B, C, and D in a checkerboard pattern by partitions 2a, and the solid cosmetic 1 is held on the tray 2 such that all of the regions A, B, C, and D are filled. Accordingly, the solid cosmetic 1 is also sectioned into four regions as well. The solid cosmetic 1 which is housed within each of the regions are of different colors, such as light blue, yellow, yellow green, pink, etc., for example. Accordingly, an upper surface 1a of the solid cosmetic 1 which is visible is in a state in which it is colored by each of the aforementioned colors.

A raised portion 1b having a predetermined pattern, here, a pattern shaped as a rose as an example, is formed on the upper surface 1a of the solid cosmetic 1. The raised portion 1b is provided over the four regions A, B, C, and D. Here, the vertical cross sectional shape of the solid cosmetic 1 in the case that the solid cosmetic 1 is viewed from the direction of arrow Y in FIG. 1 is magnified and schematically illustrated in FIG. 2. As illustrated in FIG. 2, a region of fine recesses and protrusions 1c, in which fine recesses and protrusions that include inclined surfaces are distributed two dimensionally, is formed on the surface of the raised portion. Such a raised portion 1b may be shaped by impact molding, which is a technique which is conventionally conducted on solid cosmetics. The region of fine recesses and protrusions 1c may be formed at the same time that the solid cosmetic 1 is placed on the tray 2, or may be formed after the solid cosmetic 1 is placed on the tray 2.

The region of fine recesses and protrusions 1c is formed to have the differences in height H illustrated in FIG. 2, that is, differences in height between the recesses and the protrusions, within a range from 0.1 to 10 mm. Colorants 3 having a primary particle diameter within a range from 10 to 100 µm are dispersively adhered onto the region of fine recesses and protrusions 1c. A glossy powder such as a pearly agent and a lame, which are widely employed as decorative elements for cosmetics, may be favorably employed as the colorant 3. In the case that a glossy powder is employed as the colorant, the spatial effect of the complex shape formed by the raised portion, the recesses, and the fine recesses and protrusions becomes more significant compared to a state prior to the glossy powder being adhered onto the region of fine recesses and protrusions 1c. As a result, the visibility of the solid cosmetic 1 increases, and an appearance which is more attractive to consumers can be achieved. The colorant 3 is not limited to a glossy powder, and may be particles or the like, of which the entireties or the surfaces are colored or coated by coloring pigments.

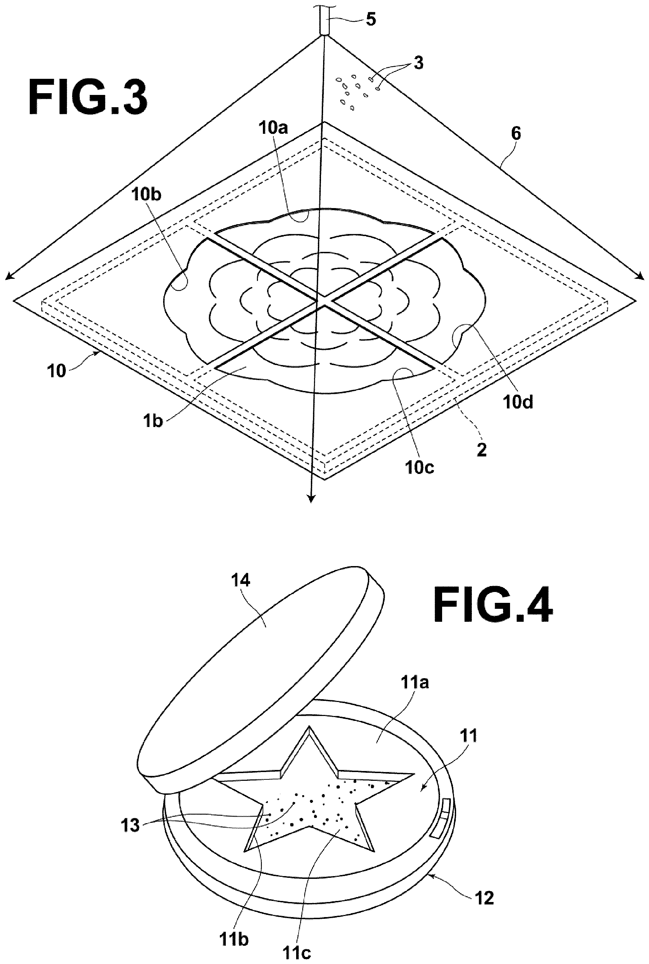

Dispersive adhesion of the colorant 3 may be conducted by applying the spray method, which is conventionally employed as a method for adding decorative elements to the surfaces of cosmetics. FIG. 3 illustrates the main portions of a configuration for executing the spray method. Hereinafter, a description will be given with reference to FIG. 3. A spray device having a spray nozzle 5 is equipped with, in addition to the spray nozzle 5, a container (not shown) for preparing a composition that contains colorants as an ink 6 to be coated by mixing particulate colorants 3 with a solvent such as alcohol, a pressure feeding device (not shown) that pressure feeds the ink 6 which is obtained, a pipe (not shown) for establishing a connection between the pressure feeding device and the spray nozzle 5, etc. The ink 6 which is pressure fed from the pressure feeding device through the pipe is sprayed out by the spray nozzle 5.

It is desirable for the viscosity of the ink 6 to be within an approximate range from 100 to 10000 mPa•s, as an example. By the viscosity of the ink 6 being within an approximate range from 100 to 10000 mPa•s, it will become possible to precisely color the upper surface of the region of fine recesses and protrusions 1c as intended, that is, without shifting, bleeding among boundaries, or the like. Although the ink 6 may include a solvent other than alcohol, for example, water, it is preferable for the ink 6 to contain greater than or equal to 50% of alcohol.

Examples of a thickener in the ink 6 include polyvinyl alcohol, polyvinyl acetate, polyvinyl methyl ether, polyvinyl pyrrolidone, a copolymer of vinyl pyrrolidone and vinyl acetate, sodium polyacrylate, poly ethyl acrylate, polyacrylic acid alkanolamine, a copolymer of alkyl methacrylate and dimethyl aminoethyl methacrylate, poly 2-acrylamide-2-methyl propane sulfonic acid, poly methacryloyl oxytrimethyl ammonium, (dimethyl acrylamide/acryloyl dimethyl taurine Na) cross polymer, etc.

When utilizing the spray device, the solid cosmetic 1 having the shaped raised portion 1b is provided at a position that faces the spray nozzle 5 in a state in which the solid cosmetic 1 is held on the tray 2. A mask 10 is placed between the upper surface 1a of the solid cosmetic 1 and the spray nozzle 5 at a position which is comparatively closer to the solid cosmetic 1. The mask has four openings 10a, 10b, 10c, and 10d which are of substantially the same shape as the raised portion 1b of the solid cosmetic 1. The mask is placed such that each of these openings 10a through 10d are aligned with the raised portion 1b. The spray device is operated after the mask 10 is placed in this manner, and the ink 6 that contains the colorant 3 is sprayed toward the solid cosmetic 1 through the spray nozzle 5.

The ink 6 which is sprayed passes only through the portions of the openings 10a, 10b, 10c, and 10d of the mask 10, and adheres to substantially the entire surface of the raised portion 1b of the solid cosmetic 1. Thereafter, the solvent within the ink 6 separates from the raised portion 1b by vaporization, air drying, or the like, and the surface of the raised portion 1b will be in a state in which only the colorant 3 remains thereon by dispersive adhesion. After the raised portion 1b is in this state, the solid cosmetic 1 is placed in a package or the like and is provided for sale.

The solid cosmetic 1 which is produced in the manner described above has the raised portion 1b having an eye catching shape on the upper surface 1a which is often viewed. The colorant 3 constituted by a pearly agent, etc. is dispersively adhered on substantially the entire surface of the raised portion 1b. Therefore, the solid cosmetic 1 has a superior visual product appeal, with a high so called eye catching effect. In the solid cosmetic 1, the region of fine recesses and protrusions has differences in height within a range from 0.1 to 10 mm and includes inclined surfaces, and the primary particle diameters of the colorant 3 which is dispersively adhered thereto is within a range from 10 to 100 µm. Therefore, an advantageous effect that the colorant 3 can be an ink in which the colorant is stably dispersed in a solvent can be achieved. In addition, another advantageous effect, that when this ink is sprayed, it is possible to precisely color the upper surface of the region of fine recesses and protrusions 1c as intended, that is, without shifting, bleeding among boundaries, or the like, can also be achieved.

The solid cosmetic 1 of the present embodiment is produced by spraying the mixed solution 6 through the mask 10 having the openings 10a through 10d as described above. Therefore, the range of dispersive adhesion of the colorant 3 is not strictly aligned with the shape of the raised portion 1b as viewed from above. That is, portions at which the colorant 3 is adhered onto the exterior of the outer edge of the raised portion 1b, and portions at the interior of the outer edge of the raised portion 1b at which the colorant 3 is not adhered are partially present.

With respect to this point, there may be cases in which it is desired for the range of dispersive adhesion of the colorant 3 to be strictly aligned with the shape of the raised portion 1b as viewed from above. Such a requirement is recognized to exist particularly in the case that the shape of the raised portion 1b as viewed from above includes many fine serrations on the outer edge thereof. In the case that this requirement is to be satisfied, the openings of the mask 10 may be cut to strictly match the shape of the raised portion 1b as viewed from above, and the positions of the openings of the mask 10 may be strictly aligned with the shape of the raised portion 1b as viewed from above when spraying the ink 6.

Locations onto which the colorant 3 is not dispersively adhered may be clearly provided as polygonal shapes or as text not only the vicinity of the outer edge of the raised portion 1b, but also within the raised portion 1b. Alternatively, locations at which the colorant 3 is dispersively adhered may be clearly provided on the upper surface 1a of outside the raised portion 1b. Such manners in which the colorant 3 is dispersively adhered on the solid cosmetic 1 are easily realizable by setting the shapes and the positions of the openings in the mask 10. The manner in which the colorant 3 is dispersively adhered onto the solid cosmetic 1 may be determined as appropriate according to the aesthetic effect to be achieved.

In the present embodiment, the colorant 3 is adhered across the entirety of the region of fine recesses and protrusions 1c, the colorant 3 may be adhered only on a portion of the region of fine recesses and protrusions 1c.

In the previous description, the raised portion 1b is provided over the four regions A, B, C, and D. Strictly speaking, the raised portion 1b is divided into four sections with the partitions 2a among the sections. Because the entirety of the raised portion 1b appears to be a single rose flower, the raised portion 1b will be perceived as extending across the four regions A through D. That is, an impression that the raised portion 1b extends over and straddles the four regions A through D. In the case that a plurality of regions of different colors are connected without the partitions 2a among them and the raised portion is formed across the regions, the raised portion 1b will be in a physically continuous state, without being divided among the regions. A raised portion 1b formed in such a manner will actually be in a state in which it extends across the plurality of regions. In the present invention, the expression "provided over" refers to both a case in which the raised portion 1b is actually continuous and a case in which the raised portion 1b appears to be continuous.

In the present embodiment, the solid cosmetic 1 is sectioned into a plurality of regions via the partitions 2a of the tray 2. Alternatively, the solid cosmetic 1 may be sectioned into a plurality of regions without the partitions 2a interposed among them. That is, the solid cosmetic 1 may be placed on the tray with a plurality of regions in a checkerboard pattern or in a vertical tricolor flag pattern, for example. If adjacent regions are colored to be different colors, each of the regions will appear different, and therefore will be in a state in which the solid cosmetic 1 is sectioned into a plurality of regions.

Next, a solid cosmetic 11 according to a second embodiment of the present invention will be described with reference to FIG. 4. The solid cosmetic 11 of the present embodiment is a foundation which is housed in a compact container. This solid cosmetic 11 is held on a container main body 12 that constitutes the compact container together with a lid 14. A recessed portion 11b having a predetermined pattern, here, a star shaped pattern as an example, is formed in an upper surface 11a of the solid cosmetic 11. A region of fine recesses and protrusions 11c that includes inclined surfaces and is constituted by fine recesses and protrusions being distributed two dimensionally is formed on a bottom surface of the recessed portion 11b. A colorant 13 which is the same as the colorant 3 described above is dispersively adhered onto the region of fine recesses and protrusions 11c.

In the present embodiment as well, the recessed portion 11b having an eye catching shape is formed in the upper surface 11a, which is often viewed, of the solid cosmetic 11. The colorant 13 constituted by a pearly agent, etc. is dispersively adhered on substantially the entire surface of the recessed portion 11b. Therefore, the solid cosmetic 11 has a superior visual product appeal, with a high so called eye catching effect. In the solid cosmetic 11, the differences in height in the region of fine recesses and protrusions 11c and the primary particle diameters of the colorant 13 which is dispersively adhered thereto are within the same ranges of numerical values as those of the first embodiment. Thereby, the same advantageous effects as those of the first embodiment are obtained.

Dispersive adhesion of the colorant 13 in the present embodiment may be conducted in the same manner as that of the first embodiment. The relationship between the range of dispersive adhesion of the colorant 13 and the shape of the recessed portion 11b as viewed from above may also be set in the same manner as the relationship between the range of dispersive adhesion of the colorant 3 and the shape of the raised portion 1b of the first embodiment.

A raised portion similar to the raised portion 1b of the first embodiment may be provided on the upper surface 11a of the solid cosmetic 11 along with the recessed portion 11b, regions of fine recesses and protrusions 11c may be formed on both the raised portion and the recessed portion 11b, and the colorant may be dispersively adhered onto both of the regions of fine recesses and protrusions 11c.

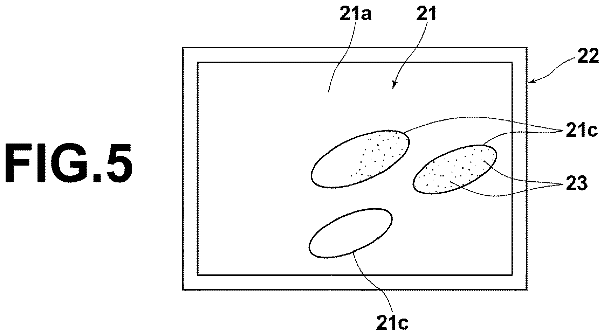

Next, a solid cosmetic 21 according to a third embodiment of the present invention will be described with reference to FIG. 5. The solid cosmetic 21 of the present embodiment is an eye shadow which is housed in a rectangular container 22. An upper surface 21a of the solid cosmetic 21 is formed to be flat. Three regions of fine recesses and protrusions 21c that include inclined surfaces and are patterned as ovals are formed on the flat upper surface 21a, and a colorant 23, which is the same as the colorant 3 described above, is dispersively adhered on the fine regions of recesses and protrusions 21c.

In the present embodiment as well, the regions of fine recesses and protrusions 21c are formed in the upper surface 21a, which is often viewed, of the solid cosmetic 21. The colorant 23 constituted by a pearly agent, etc. is dispersively adhered on substantially the entire surface of the regions of fine recesses and protrusions 21c. Therefore, the solid cosmetic 21 has a superior visual product appeal, with a high so called eye catching effect. In the solid cosmetic 21, the differences in height in the regions of fine recesses and protrusions 21c and the primary particle diameters of the colorant 13 which is dispersively adhered thereto are within the same ranges of numerical values as those of the first embodiment. Thereby, the same advantageous effects as those of the first embodiment are obtained.

Dispersive adhesion of the colorant 23 in the present embodiment may be conducted in the same manner as that of the first embodiment. The relationship between the range of dispersive adhesion of the colorant 23 and the shapes of the regions of fine recesses and protrusions 21c as viewed from above may also be set in the same manner as the relationship between the range of dispersive adhesion of the colorant 3 and the shape of the raised portion 1b as viewed from above of the first embodiment.

### [Explanation of the Reference Numerals]

- 1, 11, 21: solid cosmetic
- 1a, 2a, 3a: upper surface of solid cosmetic
- 1b: raised portion of solid cosmetic
- 1c, 11c, 21c: region of fine recesses and protrusions
- 2, 22: container
- 3, 13, 23: colorant
- 5: spray nozzle
- 6: ink
- 11b: recessed portion of solid cosmetic
- 12: container main body of compact

## Claims

1. A solid cosmetic which is held in a tray, **characterized by**:
a region of fine recesses and protrusions with differences in height within a range from 0.1 to 10 mm and that include inclined surfaces being formed on an upper surface of the solid cosmetic; and
a colorant with a primary particle diameter within a range from 10 to 100 µm is added to a portion of the upper surface of the solid cosmetic that includes at least a portion of the region of fine recesses and protrusions by dispersive adhesion.

2. The solid composition as defined in Claim 1, wherein:
the colorant is a glossy powder.

3. The solid composition as defined in either one of Claim 1 or 2, wherein:
a raised portion having a predetermined pattern is formed on the upper surface of the solid cosmetic; and
the region of fine recesses and protrusions is formed at least on a portion of a surface of the raised portion.

4. The solid composition as defined in any one of Claims 1 through 3, wherein:
a recessed portion having a predetermined pattern is formed on the upper surface of the solid cosmetic; and
the region of fine recesses and protrusions is formed at least on a portion of a surface of the recessed portion.

5. The solid cosmetic as defined in either one of Claim 1 or 2, wherein:
the upper surface of the solid cosmetic is formed to be flat; and
the region of fine recesses and protrusions is formed on the flat upper surface.

6. The solid cosmetic as defined in any one of Claims 1 through 5, wherein:
the upper surface is sectioned into a plurality of regions; and
the solid cosmetic is colored to be a different color in each of the regions.

7. The solid cosmetic as defined in Claim 6; wherein:
the region of fine recesses and protrusions is provided over the plurality of regions.

8. A method for producing the solid cosmetic according to any one of Claims 1 through 6, **characterized by** comprising:
placing a solid cosmetic having a region of fine recesses and protrusions with differences in height within a range from 0.1 to 10 mm and that include inclined surfaces an upper surface thereof on a tray, and thereafter;
dispersing a composition that contains a colorant with a primary particle diameter within a range from 10 to 100 µm onto at least the region of fine recesses and protrusions formed on the upper surface of the solid cosmetic.

9. The method for producing the solid cosmetic as defined in Claim 8, wherein:
the composition is dispersed onto the upper surface of the solid cosmetic via a mask having an opening that corresponds to a range onto which the colorant is to be dispersively adhered.
